# EUROPEAN PATENT SPECIFICATION

(11) **EP 2 349 251 B1**
(45) Date of publication and mention of the grant of the patent: **06.03.2013**
(21) Application number: 09793308.9
(22) Date of filing: 06.10.2009
(51) Int. Cl.: A61K 31/31, A61K 9/00

(54) **TOPICAL FORMULATIONS WITH A TERTIARY AMINE OXIDE**
TOPISCHE FORMULIERUNGEN MIT EINEM TERTIÄREN AMINOXID
FORMULATIONS TOPIQUES AVEC UN OXYDE D'AMINE TERTIAIRE

(30) Priority: 06.10.2008 US 102975 P; 18.08.2009 US 542739
(43) Date of publication of application: 03.08.2011
(73) Proprietor: Bausch & Lomb Incorporated, Rochester, NY 14604-2701 (US)
(72) Inventor: MARLOWE, Zora, Teresa, Rochester NY 14616 (US); BANDYOPADHYAY, Paramita, Webster NY 14580 (US); WANG, Hongna, Fairport NY 14450 (US); PHILLIPS, Eric, Ontario NY 14519 (US)
(74) Representative: Glas, Holger
(86) International application number: PCT/US2009/059605
(87) International publication number: WO 2010/042459

(56) References cited:
- WO-A-2008/049042
- JP-A- 2007 106 727
- US-A1- 2007 015 710
- US-A1- 2008 194 518

## Description

The present invention relates to formulations that include a pharmaceutical active and a tertiary amine oxide.

### Background of the Invention

An ophthalmic formulation such as an eye drop formulation that contains a pharmaceutical active typically includes a preservative agent to inhibit growth of bacteria and/or fungi if the formulation becomes contaminated with such organisms. Various preservative agents are known for use in ophthalmic formulations. Such preservative agents should have a broad spectrum of preservative activity and be non-irritating to the eye. Many preservative agents, however, have a tendency to irritate eye tissue, especially if present at relatively high concentrations. Accordingly, ophthalmic formulations that contain relatively small amounts of preservative agent, as described in WO2008/049042, or a preservative agent with a relatively low toxicity profile, are of continued interest.

JP 2007/106727 describes ophthalmical pharmaceutical formulations that contain PEG-type amino oxides as well as tertiary amine oxides. However, JP 2007/106727 discloses merely to use the amine oxides as a cosmetic component and, furthermore, in very high concentrations from 0.1 to 30 percent by weight (1000 ppm to 300000 ppm). Also US 2008/194518 and US 2007/015710 describe ophthalmic formulations that contain tertiary amine oxides in very high amounts.

U.S. Patent No. 5,840,671 describes a contact lens solution that contains a tertiary amine oxide and an anionic surfactant in the form of a triethanolamine salt. These two cleaning components are present in the solution at a total concentration from 0.1 wt.% to 20 wt.%. Also the weight ratio of the tertiary amine oxide to the anionic surfactant is from 1:4 to 30:1. It is said that the combination of the tertiary amine oxide with the anionic surfactant provide a "higher degree of cleaning effect than any cleaning solution which employs only one of those two kinds of surface active agents." Also, if the total amount of tertiary amine oxide and anionic surfactant is less than 0.1 wt.%, the lens solution "does not exhibit a satisfactory cleaning effect".

### Summary of the Invention

The invention is directed to a topical pharmaceutical formulation comprising 0.5 ppm to 60ppm of a tertiary amine oxide of general formula I 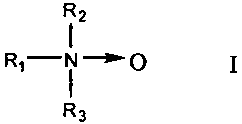 wherein R₁ is a C₈-C₁₈alkyl, and R₂ and R₃ are independently selected from a C₁-C₄alkyl or C₁-C₄hydroxyalkyl and a pharmaceutical active agent. In particular, the tertiary amine oxide functions as a preservative or enhances the preservative efficacy in such formulations.

The topical pharmaceutical formulations furthermore comprise an additional preservative component, wherein the preservative component is a cationic, preservative component selected from the group consisting of quaternary ammonium compounds and their polymers and biguanides and their polymers, wherein the formulation is a preserved ophthalmic composition.

The ophthalmic composition will also have an osmolality of 225 mOsm/kg to 400 mOsm/kg.

### Detailed Description of the Invention

As used herein, the term "pharmaceutical active agent" refers to a compound, or a mixture of compounds, that when administered to a subject (human or animal) causes a desired pharmacologic and/or physiologic effect by local and/or systemic action. Accordingly, pharmaceutical formulations comprising a tertiary amine oxide of general formula I have the benefit of being adequately preserved without having a harsh physiological affect such as irritation or discomfort, which is common with many preservative agents. The term "prescription pharmaceutical agent is a pharmaceutical active agent that is included in a formulation in which the formulation requires physician approval (a physician prescription) by the U.S. food and Drug Administration.

The invention is directed to a topical pharmaceutical formulation comprising: a tertiary amine oxide of general formula I 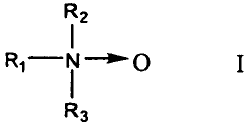 wherein R₁ is a C₈-C₁₈alkyl, and R₂ and R₃ are independently selected from a C₁-C₄alkyl or C₁-C₄hydroxyalkyl; and a pharmaceutical active agent , wherein the tertiary amine oxide functions as a preservative or enhances the preservative efficiency of the pharmaceutical formulation, and an additional preservative component, wherein the preservative component is a cationic, preservative component selected from the group consisting of quaternary ammonium compounds and their polymers and biguanides and their polymers, wherein the formulation is a preserved ophthalmic composition having an osmolality of 225 mOsm/kg to 400 mOsm/kg. The invention is also directed to the use of a tertiary amine oxide of general formula I to preserve a topical pharmaceutical formulation, particularly an ophthalmic formulation , that includes a pharmaceutical agent and an additional preservative component 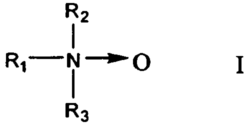 wherein R₁ is a C₈-C₁₈Alkyl, and R₂ and R₃ are independently selected from a C₁-C₄alkyl or C₁-C₄hydroxyalkyl, the tertiary amin oxide is present from 0.5 ppm to 60 ppm, the preservative component is a cationic, preservative component selected from the group consisting of quaternary ammonium compounds and their polymers and biguanides and their polymers, the formulation having an osmolality of 225 mOsm/kg to 400 mOsm/kg.

The tertiary amine oxide of general formula I is present in the formulation from 0.5 ppm to 60 ppm.

The presence of the tertiary amine oxide of general formula I in the described topical pharmaceutical formulations demonstrates a preservative "synergy" with a variety of cationic preservative components. Accordingly, the tertiary amine oxide is present as a co-preservative component with another preservative component. The tertiary amine oxide is believed to complement a preservative component in the formulation. In fact, relatively small amounts of tertiary amine oxide are needed to enhance the preservative effectiveness of the formulations, particularly against fungi, e.g., *Fusarium solani* and *Candida albicans.* Typically, formulations that include a tertiary amine oxide of general formula I exhibit a reduction in the cell population by two log orders after 7 days of the five following microorganisms, *Staphylococcus aureus, Pseudomonas aeruginosa, Eschrechia coli, Candida albicans* and *Aspergillus niger,* or will prevent the growth of fungal bioburden by ±0.5 log. See and compare, for example, the stand-alone biocidal data listed in Tables 7 and 8.

As stated, the topical formulations also include an additional preservative component. The preservative component is selected from quaternary ammonium compounds (including small molecules) and polymers and low and high molecular weight biguanides. For example, biguanides include the free bases or salts of alexidine, chlorhexidine, hexamethylene biguanides and their polymers (PHMB) and any one mixture thereof. The salts of alexidine and chlorhexidine can be either organic or inorganic and include gluconates, nitrates, acetates, phosphates, sulfates, halides and the like.

In a preferred embodiment, the formulation will include a polymeric biguanide known as poly(hexamethylene biguanide) (PHMB or PAPB) commercially available from Arch Chemical under Cosmocil^{®}CQ. The PHMB is present in the formulations from 0.1 ppm to 1.3 ppm. Another biguanide of interest is 1,1'-hexamethylene-bis[5-(2-ethylhexyl)biguanide], which is referred to in the art as "alexidine". The alexidine is present in a solution from 0.5 ppm to 5 ppm or from 0.5 ppm to 2 ppm.

One of the more common quaternary ammonium compounds is α-[4-tris(2-hydroxyethyl)-ammonium chloride-2-butenyl]poly[1-dimethyl ammonium chloride-2-butenyl]-ω-tris(2-hydroxyethyl) ammonium chloride, also referred to in the art as polyquaternium-1. Quaternary ammonium compounds are generally referred to in the art as "polyquaternium" disinfectants, and are identified by a particular number following the designation such as polyquaternium-1, polyquaternium-10 or polyquaternium-42. Polyquaternium-1 is present in the formulations from 0.5 ppm to 3 ppm. Polyquaternium-42 is also one of the more preferred polyquaternium disinfectants, see, U.S. Patent No. 5,300,296. Polyquaternium-42 is present in the formulations from 5 ppm to 50 ppm.

Still another preservative component that is used in combination with a tertiary amine oxide of general formula I is polylysine.

In some ophthalmic formulations such as a multi-dose rewet drop for use with contact lenses or a formulation to alleviate ocular discomfort or dye eye symptoms, the formulations can include one or more demulcents, comfort or cushioning components. Suitable comfort components include, but are not limited to, water soluble natural gums, cellulose-derived polymers and the like and dexpanthenol. Useful natural gums include guar gum, gum tragacanth and hydroxypropyl guar. Useful cellulose-derived comfort components include cellulose-derived polymers, such as hydroxypropyl cellulose, hydroxypropylmethyl cellulose, carboxymethyl cellulose, methyl cellulose, hydroxyethyl cellulose and the like. A very useful comfort component is hydroxypropylmethyl cellulose (HPMC). Some non-cellulose comfort components include propylene glycol or glycerin. The comfort components are typically present in the solution from 0.01 % to 1% (w/v).

One preferred comfort agent is hyaluronic acid, which is a linear polysaccharide (long-chain biological polymer) formed by repeating disaccharide units consisting of D-glucuronic acid and N-acetyl-D-glucosamine linked by β(1-3) and β(1-4) glycosidic linkages. Hyaluronic acid is distinguished from the other glycosaminoglycans, as it is free from covalent links to protein and sulphonic groups. Hyaluronic acid is ubiquitous in animals, with the highest concentration found in soft connective tissue. It plays an important role for both mechanical and transport purposes in the body; e.g., it gives elasticity to the joints and rigidity to the vertebrate disks, and it is also an important component of the vitreous body of the eye.

Hyaluronic acid is accepted by the ophthalmic community as a compound that can protect biological tissues or cells from compressive forces. Accordingly, hyaluronic acid has been proposed as one component of a viscoelastic ophthalmic composition for cataract surgery. The viscoelastic properties of hyaluronic acid, that is, hard elastic under static conditions though less viscous under small shear forces enables hyaluronic acid to basically function as a shock absorber for cells and tissues. Hyaluronic acid also has a relatively large capacity to absorb and hold water. The stated properties of hyaluronic acid are dependent on the molecular weight, the solution concentration, and physiological pH. At low concentrations, the individual chains entangle and form a continuous network in solution, which gives the system interesting properties, such as pronounced viscoelasticity and pseudoplasticity that is unique for a water-soluble polymer at low concentration.

Another comfort agent of specific interest is alginate. Alginate is an anionic biopolymer produced by a variety of microorganisms and marine algae. Alginate is a polysaccharide that comprises β-D-mannuronic acid units and α-L-guluronic acid units. Some alginate polymers are block copolymers with blocks of the guluronic acid (or salt) units alternating with blocks of the mannuronic acid (or salt) units as depicted in-part below. Some alginate molecules have single units of guluronic acid (or salt) alternating with single units of mannuronic acid (or salt). The ratio and distribution of the mannuronic and guluronic unit, along with the average molecular weight, affect the physical and chemical properties of the copolymer. See Haug, A. et al., Acta Chem. Scand., 183-90 (1966). Alginate polymers have viscoelastic rheological properties and other properties that make it suitable for some medical applications. See Klock, G. et al., "Biocompatibility of mannuronic acid-rich alginates," Biomaterials, Vol. 18, No. 10, 707-13 (1997). The use of alginate as a thickener for topical ophthalmic use is disclosed in U.S. Patent No. 6,528,465 and U.S. Patent Application Publication 2003/0232089. In U.S. Patent No. 5,776,445, alginate is used as a drug delivery agent that is topically applied to the eye. U.S. Patent Publication No. 2003/0232089 teaches a dry-eye formulation that contains two polymer ingredients including alginate.

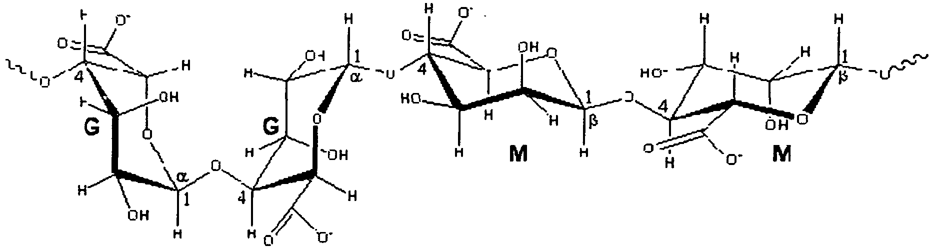

The alginate used in the formulations will typically have a number average molecular weight from about 20 kDa to 2000 kDa, or from about 100 kDa to about 1000 kDa, for example about 325 kDa. The concentration of alginate is from about 0.01 wt.% to about 2.0 wt.%. More, typically, the concentration of alginate is from about 0.1 wt.% to about 0.5 wt.%.

In some instances, the topical formulation will include a prescription pharmaceutical agent. Also, as mentioned, the topical formulation can be an ophthalmic formulation prescribed by or recommended by a physician, or a health care provider, e.g., an O.D., for the treatment of an ocular condition or an ocular disease. Likewise, the ophthalmic formulation can also include a prescription pharmaceutical agent.

Exemplary ophthalmic formulations for the treatment of dry eye are provided in Tables 1 to 5. Each component is listed as % w/w except as noted and the alkylamine oxide is myristamine oxide, lauramine oxide or any mixture thereof. Additional information on dry eye formulations can be found in U.S. patent application serial no. 11/842,394, filed August 21, 2007, now U.S. Publication No. 2008/0057022.

**Table 1.**

| **Component** | **% w/w** |
|---|---|
| Carbopol® 980NF | 0.02 to 0.2 |
| glycerin | 0.01 to 0.5 |
| alkylamine oxide | 0.001 to 0.006 |
| PHMB (ppm) | 0.3 to 1.1 |
| sorbitol | 0.5 to 5.0 |
| purified water | q.s. to 100% |

Other ophthalmic formulations of a sterile, buffered, hypotonic solution intended for use as an artificial tear and lubricant for providing soothing therapy to dry irritated eyes are listed in Tables 2 to 5. For example, the solutions of Table 2 are non-blurring, low viscosity solutions that contains propylene glycol and glycerin as demulcents, which are believed to lubricate or cushion the corneal epithelium. The solution also contains alginate, a hydrocolloid, which is believed to interact with the mucin layer in the tear film and maintain a moist ocular environment for an extended period of time. The alginate is also believed to stabilize the tear film and provide long term relief to dry eyes.

**Table 2.**

| **Component** | **% w/w** |
|---|---|
| Protanal LF200M alginate | 0.05 to 0.5 |
| glycerin | 0.1 to 1.0 |
| propylene glycol | 0.1 to 1.0 |
| alkylamine oxide | 0.001 to 0.006 |
| PHMB (ppm) | 0.3 to 0.8 |
| sodium borate | 0.01 to 0.04 |
| boric acid | 0.2 to 0.8 |
| Dequest®2016 | 0.02 to 1.2 |
| purified water, USP | Q.S. to 100% |

**Table 3.**

| **Component** | **% w/w** |
|---|---|
| hydroxyethyl cellulose or | 0.2 to 2.0 |
| hydroxypropyl guar | |
| propylene glycol | 2.0 to 20 |
| alkylamine oxide | 0.001 to 0.006 |
| Polyquaternium-1 (ppm) | 1 to 5 |
| EDTA | 0.02 to 0.25 |
| purified water, USP | Q.S. to 100% |

**Table 4.**

| **Component** | **% w/w** |
|---|---|
| mineral oil | 3.0 to 7.0 |
| phosphate buffer^{a} | 0.1 to 0.5 |
| alkylamine oxide | 0.001 to 0.006 |
| PHMB (ppm) | 0.3 to 0.8 |
| EDTA | 0.005 to 0.02 |
| surfactant ^{b} | 0.2 to 1.0 |
| NaCl | 0.3 to 1.0 |
| purified water, USP | Q.S. to 100% |

| | |
|---|---|
| a - A mixture of NaHPO₄ and Na₂PO₄. b - A mixture of polysorbate 80 and PEG-40-octylphenyether | |

**Table 5.**

| **Component** | **% w/w** |
|---|---|
| hyaluronate | 0.05 to 0.25 |
| phosphate buffer^{a} | 0.1 to 0.5 |
| alkylamine oxide | 0.001 to 0.006 |
| glycerin | 0.3 to 1.2 |
| EDTA | 0.005 to 0.02 |
| NaCl | 0.3 to 1.0 |
| purified water, USP | Q.S. to 100% |

| | |
|---|---|
| a - A mixture of NaHPO₄ and Na₂PO₄. | |

Again, the topical formulations also include a cationic preservative component.

An comparative Exemplary set of ophthalmic formulations with amino alcohol/zinc salt preservative system are provided in Table 6.

**Table 6.**

| **Component** | **% w/w** |
|---|---|
| hydroxypropyl guar | 0.1 to 0.6 |
| borate acid | 0.2 to 0.7 |
| alkylamine oxide | 0.001 to 0.1 |
| ZnCl | 0.3 to 1.2 |
| AMP | 0.05 to 0.5 |
| propylene glycol | 0.5 to 2.0 |
| purified water, USP | Q.S. to 100% |

### 1. Use of one or more alkyl amineoxides in a a topical pharmaceutical formulation.

The pharmaceutical active agent can be any compound that is used to treat any one disease or any one medical condition. Accordingly, the pharmaceutical active agent can be selected from any one class of compounds, for example, anti-inflammatory agents, anti-infective agents (including antibacterial, antifungal, antiviral, antiprotozoal agents), anti-allergic agents, antiproliferative agents, anti-angiogenic agents, anti-oxidants, antihypertensive agents, neuroprotective agents, cell receptor agonists, cell receptor antagonists, immunomodulating agents, immunosuppressive agents, IOP lowering agents, beta adrenoceptor antagonists, alpha-2 adrenoceptor agonists, carbonic anhydrase inhibitors, cholinergic agonists, prostaglandins and prostaglandin receptor agonists, angiotensin converting enzyme ("ACE") inhibitors, AMPA receptor antagonists, NMDA antagonists, angiotensin receptor antagonists, somatostatin agonists, mast cell degranulation inhibitors, alpha-adrenergic receptor blockers, alpha-2 adrenoceptor antagonists, thromboxane A2 mimetics, protein kinase inhibitors, prostaglandin F derivatives, prostaglandin-2 alpha antagonists, cyclooxygenase-2 inhibitors and muscarinic agents.

Of particular interest are pharmaceutical active agents that are known to treat an ocular disease or disorder including, but are not limited to, a posterior-segment disease or disorder. In certain embodiments, such ocular disease or disorder is selected from the group consisting of diabetic retinopathy, diabetic macular edema, cystoid macular edema, age macular degeneration (including the wet and dry form), optic neuritis, retinitis, chorioretinitis, intermediate and posterior uveitis and choroidal neovascuralization.

Glaucoma is a group of diseases that are characterized by the death of retinal ganglion cells ("RGCs"), specific visual field loss, and optic nerve atrophy. Glaucoma is the third leading cause of blindness worldwide. An intraocular pressure ("IOP") that is high compared to the population mean is a risk factor for the development of glaucoma. However, many individuals with high IOP do not have glaucomatous loss of vision. Conversely, there are glaucoma patients with normal IOP. Therefore, continued efforts have been devoted to elucidate the pathogenic mechanisms of glaucomatous optic nerve degeneration.

It has been postulated that optic nerve fibers are compressed by high IOP, leading to an effective physiological axotomy and problems with axonal transport. High IOP also results in compression of blood vessels supplying the optic nerve heads ("ONHs"), leading to the progressive death of RGCs. See; e.g., M. Rudzinski and H.U. Saragovi, Curr. Med. Chem.-Central Nervous System Agents, Vol. 5, 43 (2005).

In one embodiment, the anti-glaucoma pharmaceutical agent is of general formula II 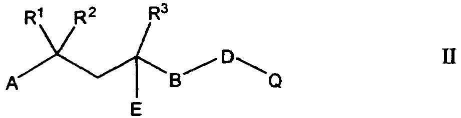 wherein A and Q are independently selected from the group consisting of aryl and heteroaryl groups substituted with at least a halogen atom, cyano group, hydroxy group, or C₁-C₁₀ alkoxy group; R¹, R², and R³ are independently selected from the group consisting of unsubstituted and substituted C₁-C₅ alkyl groups; B is a C₁-C₅ alkylene group; D is the NH- or -NR'- group, wherein R' is a C₁-C₅ alkyl group; and E is the hydroxy group.

Exemplary, pharmaceutical agents of general formula II include A as a dihydrobenzofuranyl group substituted with a fluorine atom; Q as a quinolinyl or isoquinolinyl group substituted with a methyl group; R¹ and R² are independently selected from the group consisting of unsubstituted and substituted C₁-C₅ alkyl groups; B is a C₁-C₃ alkylene group; D is the -NH- group; E is a hydroxy group; and R³ is a trifluoromethyl group.

Exemplary compounds include a glucocorticoid receptor agonist having Formulae III or IV, as disclosed in US Patent Application Publication 2006/0116396. 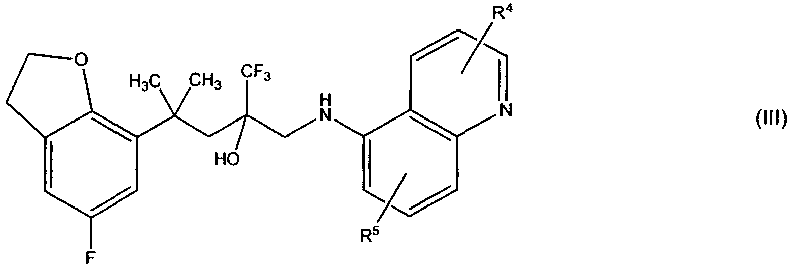 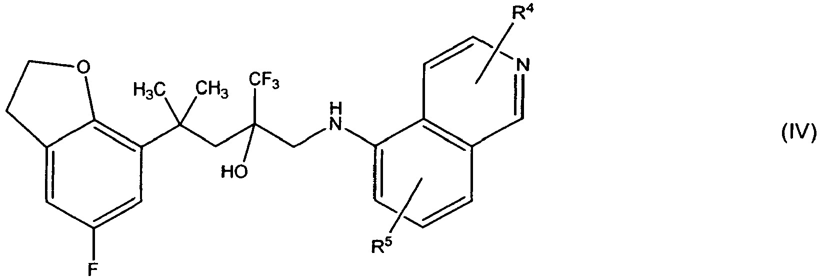 wherein R⁴ and R⁵ are independently selected from the group consisting of hydrogen, halogen, cyano, hydroxy, C₁-C₁₀ (alternatively, C₁-C₅ or C₁-C₃) alkoxy groups, unsubstituted C₁-C₁₀ (alternatively, C₁-C₅ or C₁-C₃) linear or branched alkyl groups, substituted C₁-C₁₀ (alternatively, C₁-C₅ or C₁-C₃) linear or branched alkyl groups, unsubstituted C₃-C₁₀ (alternatively, C₃-C₆ or C₃-C₅) cyclic alkyl groups, and substituted C₃-C₁₀ (alternatively, C₃-C₆ or C₃-C₅) cyclic alkyl groups.

Another embodiment includes ocular formulations prescribed by or recommended by a physician, or a health care provider, to treat an ocular allergic conditions. Allergy is characterized by a local or systemic inflammatory response to allergens. Allergic conjunctivitis is a disorder that is characterized by the clinical signs and symptoms of eye itching, redness, tearing, and swelling. An estimated 20% of the population in the United States suffer from inflammation of the eye. The signs and symptoms of allergic conjunctivitis can significantly impact the quality of life of patients, from social interactions, productivity at work and school, to the ability to perform visual tasks such as working on a computer or reading.

Currently, available pharmaceutical treatments for inflammation of the eye or symptoms of inflammation of the eye include (1) antihistamines, (2) drugs that block the release of histamine and other substances from the mast cell (e.g., mast cell stabilizers), (3) drugs with multiple modes of action (e.g. antihistamine/mast cell stabilizing agents), and (4) drugs that can actively constrict blood vessels thus reducing redness and swelling (e.g., vasoconstrictors). Additionally, artificial tears have been used to wash the eye of allergens.

The desirability of a particular treatment tor inflammation of the eye can be measured against the following factors (1) efficacy at onset of action, (2) duration of action, (3) efficacy at controlling signs and symptoms of allergic conjunctivitis, and (4) comfort of the drop when instilled in the eye.

In still another aspect, the formulation comprising: (a) ketotifen or a salt thereof in a concentration of from a 0.001 % to 0.2% (weight/volume or "w/v"); (b) naphazoline or a salt thereof in a concentration of from 0.001% to 0.2% (w/v); and (c) water.

Ketotifen or any ophthalmically acceptable ketotifen salt may be used in the method herein described, although ketotifen fumarate is preferred. Ketotifen fumarate is represented by the following formula:

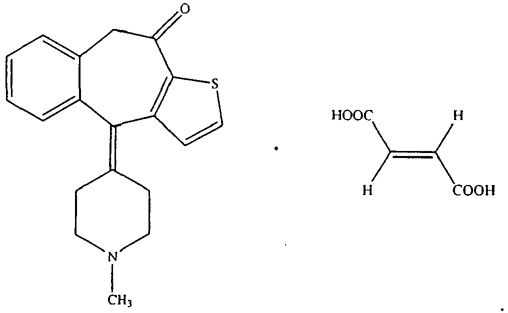

Ketotifen or a ketotifen salt is present in a formulation in a concentration from 0.001 % to 0.2% (or alternatively, from 0.001% to 0.1%). In one embodiment, ketotifen or a ketotifen salt is present in a concentration from 0.0 1 % to 0.05%; preferably, from 0.0 1 % to 0.04%; more preferably, from 0.02% to 0.03%. Concentrations of ketotifen salts yielding such concentrations of ketotifen may be readily calculated; for example, using ketotifen fumarate in a concentration of 0.0345% provides a concentration of ketotifen in the formulation of 0.025%.

Another embodiment is directed to an ocular formulation that includes an anti-redness agent, which may relieve redness in the eye. The preferred anti-redness agent is naphazoline or an ophthalmically acceptable salt thereof such as, for example, naphazoline hydrochloride. Other anti-redness agents that may be used include, but are not limited to, tetrahydrozoline, ephedrine, phenylephrine, oxymetazoline, xylometazoline, pseudoephedrine, tramazoline, other vasoconstrictors, combinations thereof, as well as ophthalmically acceptable salts thereof (e.g., tetrahydrozoline hydrochloride).

Naphazoline hydrochloride is represented by the following formula:

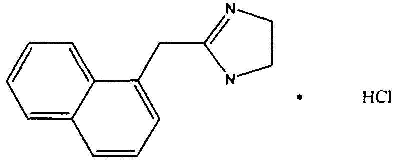

Naphazoline or a naphazoline salt may be present in a composition produced a method of the present invention in a concentration from 0.001 % to 0.2% (or alternatively, from 0.00 1 % to 0.1%). In one embodiment, naphazoline or a naphazoline salt is present in a composition at a concentration from 0.0 1 % to 0.1 %; preferably, from 0.0 1 % to 0.07%; more preferably, from 0.02% to 0.06%. Concentrations of a naphazoline salt yielding such concentrations of naphazoline base may be readily calculated; for example, using naphazoline hydrochloride in a concentration of about 0.025% provides a concentration of naphazoline base in the formulation of 0.021 %. Additional information on formulations containing ketotifen, naphazoline or a corresponding pharmaceutically salt of each thereof can be found in U.S. patent application serial no. 10/972,571,filed October 25, 2005.

Ophthalmic compositions will typically include tonicity agents, e.g., salts such as NaCl, to approximate the osmotic pressure of normal lachrymal fluids, which, as stated in U.S. Patent No. 6,274,626, is equivalent to a 2.5% solution of glycerol. Osmotic pressure, measured as osmolality, is generally about 180 to 420 mOsm/kg for conventional ophthalmic formulations.

In particular, ophthalmic formulations according to claim 1 have a measured osmolality from 225 to 400 mOsm/kg.

Another embodiment is directed to a pharmaceutical formulation prescribed by or recommended by a physician, or a health care provider, to treat a dermatological condition or a dermatological disease. For example, it is known that compounds of the FK506 class can be formulated into stable emulsions. Emulsions, since they contain an aqueous phase, are much less occlusive than oil-based compositions and hence are better tolerated in many situations. Accordingly, in one embodiment a topical formulation, in the form of an emulsion, comprises a compound of the FK506 class, a physiologically acceptable alkanediol, ether diol or diether alcohol containing up to 8 carbon atoms as solvent for the compound of the FK506 class and one or more alkyl amineoxides as a preservative agent. A compound of the "FK506 class" is a compound which has the basic structure as FK506 and which has at least one of the biological properties of FK506 (e.g., immunosuppressant properties). The compound may be in free base form or pharmaceutically acceptable, acid addition, salt form. A preferred compound of the FK 506 class is disclosed in EP 427 680, e.g. Example 66a (also called 33-epi-chloro-33-desoxyascomycin).

### Examples

### Example 1

A pharmaceutical formulation is prepared in accordance with the components listed in Table 7

**Table 7**

| **Component** | **%w/w** |
|---|---|
| betaxolol HCl | 0.5 |
| NaCl | 0.1 |
| Polyquaternium-1 | 0.001 |
| myristamine oxide | 0.005 |
| EDTA | 1.0 |
| purified water, USP | 98 |

### Comparative Examples C1, C2, C3 and C4

The following borate buffered formulations were prepared and are reported in Table 8 Each formulation includes 0.5 wt.% boric acid and 0.014 wt.% sodium borate along with the other components listed. The preservative efficacy data at 14 and 28 days is also reported in Table 8 for each of the Example formulations. The C1, C4, C2A and C3A data is the initial PE data, and the C2B and C3B data is the PE data following storage in poly(ethylene terephalate) (PET) bottles for four months at 40 °C. As indicated the preservative effectiveness of the formulations is maintained upon storage under relatively high temperature conditions.

More importantly, however, is the significant degree of preservative efficacy observed in the sodium alginate solutions. In prior attempts to develop ophthalmic formulations that include both sodium alginate and PHMB, Applicants and others at Bausch & Lomb observed a dramatic fall off in preservative effectiveness of the formulations. One suspected some form of complexation between the cationic PHMB and the anionic alginate that would essentially tie-up the PHMB. The presence of the alkyl amine oxide somehow mitigates this complexation, and quite surprisingly, the preservative efficacy of the formulations are maintained even after four months at 40 °C.

**Table 8**

| **Example** | **C1** | **C2** | | **C3** | | **C4** |
|---|---|---|---|---|---|---|
| Na alginate ^{a} | 0.25 | 0.25 | | 0..25 | | 0.25 |
| PHMB (ppm) | 1.0 | 1 | | 2.0 | | 2.0 |
| lauramine oxide (ppm) | -- | 100 | | 100 | | -- |
| glycerin | 0.6 | 0.6 | | 0.6 | | 0.6 |
| propylene glycol | 0.6 | 0.6 | | 0.6 | | 0.6 |
| Dequest®2016 | 0.05 | 0.05 | | 0.05 | | 0.05 |

| **USP PE Test (days)** | **C1** | **C2A** | **C2B** | **C3A** | **C3B** | **C4** |
|---|---|---|---|---|---|---|
| S. Aureus (14) | 3.7 | >4.7 | >4.8 | >4.7 | >4.8 | >4.7 |
| (28) | >4.7 | >4.7 | >4.8 | >4.7 | >4.8 | >4.7 |
| P. Aeruginosa (14) | 1.6 | 4.7 | 4.5 | >4.7 | >4.8 | 1.6 |
| (28) | 0.7 | >4.7 | >4.8 | >4.7 | >4.8 | 0.8 |
| E. Coli (14) | 1.6 | 3.3 | 2.2 | >4.7 | >4.8 | 2.4 |
| (28) | 0.8 | >4.7 | >4.8 | >4.7 | >4.8 | 3.6 |
| C. Albicans (14) | 0.7 | >48 | >5.0 | >4.8 | >5.0 | 0.8 |
| (28) | 2.5 | >4.8 | >5.0 | >4.8 | >5.0 | 3.0 |
| A. Niger (14) | 1.2 | >4.7 | 4.7 | 4.1 | >4.7 | 1.2 |
| (28) | 1.2 | >4.7 | >4.7 | >4.7 | >4.7 | 1.1 |

| | | | | | | |
|---|---|---|---|---|---|---|
| a Protanal LF200M | | | | | | |

Aqueous ophthalmic formulations, which can be used as a contact lens rewet eye drop formulation, are provided in Table 9

**Table 9**

| **Component (wt.%)** | **C5** | **2** | **C6** | **C7** | **C8** |
|---|---|---|---|---|---|
| sodium hyaluronate | 0.1 | 0.1 | 0.1 | 0.1 | 0.1 |
| sodium chloride | 0.42 | 0.42 | 0.42 | 0.42 | 0.42 |
| potassium chloride | 0.14 | 0.14 | 0.14 | 0.14 | 0.14 |
| boric acid | 0.6 | 0.6 | 0.6 | 0.6 | 0.6 |
| sodium borate | 0.035 | 0.035 | 0.035 | 0.035 | 0.035 |
| calcium chloride | 0.006 | 0.006 | 0.006 | 0.006 | 0.006 |
| magnesium chloride | 0.006 | 0.006 | 0.006 | 0.006 | 0.006 |
| lauramine oxide (ppm) | 50 | 50 | 50 | 50 | -- |
| stabilized oxy-chloride (purite) | 0.003 | -- | -- | -- | 0.003 |
| PHMB (ppm) | -- | 0.8 | -- | | -- |
| sorbic acid | -- | -- | 0.005 | | -- |
| Na perborate (ppm) | -- | -- | -- | 400 | -- |

### Examples 3 to 5

Aqueous ophthalmic formulations, which can be used as a contact lens rewet eye drop formulation, are provided in Table 10

**Tablet 10**

| **Component (wt.%)** | **3** | **4** | **5** | **C9** |
|---|---|---|---|---|
| hydroxypropyl guar gum | 0.15 | 0.1 | 0.15 | 0.15 |
| HPMC E4M | 0.3 | 0.2 | -- | 0.3 |
| hyaluronic acid | -- | 0.1 | 0.2 | -- |
| boric acid | 0.8 | 0.8 | 0.8 | 0.8 |
| sodium borate | 0.035 | 0.035 | 0.035 | 0.035 |
| propylene glycol | 0.8 | 0.8 | 0.8 | 0.8 |
| glycerol | 0.3 | 0.3 | 0.03 | 0.3 |
| lauramine oxide (ppm) | 50 | 50 | 50 | -- |
| polyquaternium-1 (ppm) | 1 | 1 | 1 | 1 |

## Claims

1. A topical pharmaceutical formulation comprising: 0.5 ppm to 60 ppm of a tertiary amine oxide of general formula I 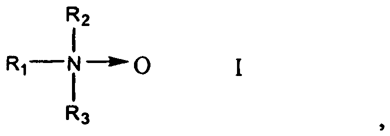 wherein R₁ is a C₈-C₁₈alkyl, and R₂ and R₃ are independently selected from a C₁-C₄alkyl or C₁-C₄hydroxyalkyl; a pharmaceutical active agent, wherein the tertiary amine oxide functions as a preservative or enhances the preservative efficacy of the pharmaceutical formulation, and an additional preservative component, wherein the preservative component is a cationic, preservative component selected from the group consisting of quaternary ammonium compounds and their polymers and biguanides and their polymers, wherein the formulation is a preserved ophthalmic composition having an osmolality of 225 mOsm/kg to 400 mOsnVkg.

2. The formulation of claim 1, wherein the tertiary amine oxide includes myristamine oxide or lauramine oxide.

3. The formulation of claims 1 or 2 further comprising a comfort component selected from the group consisting of hydroxypropyl guar, hyaluronic acid, alginate, dexpanthenol and hydroxypropylmethyl cellulose.

4. The formulation of claim 1, wherein the cationic, preservative component is selected from poly(hexamethylene biguanide), which is present in the formulation from 0.1 ppm to 1.3 ppm, alexidine, which is present in the formulation from 0.5 ppm to 5 ppm, or α-[4-tris(2-hydroxyethyl)-ammonium chloride-2-butenyl]poly[1-dimethyl ammonium chloride-2-butenyl]-ω-tris(2-hydroxyethyl) ammonium chloride (polyquaternium-1), which is present in the formulation from 0.5 ppm to 3 ppm, or polyquaternium-42.

5. The formulation of any one of claims 1, 3 and 4, wherein the preservative component is polylysine.

6. The formulation of any one of claims 1 to 5, wherein the pharmaceutical agent is a prescription pharmaceutical agent.

7. The use of a tertiary amine oxide of general formula I to contribute to the preservation of a topical ophthalmic formulation that includes a pharmaceutical agent and an additional preservative component 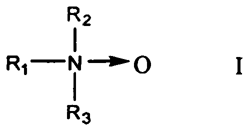 wherein R₁ is a C₈-C₁₈alkyl, and R₂ and R₃ are independently selected from a C₁-C₄alkyl or C₁-C₄hydroxyalkyl, the tertiary amine oxide is present from 0.5 ppm to 60 ppm, the preservative component is a cationic, preservative component selected from the group consisting of quaternary ammonium compounds and their polymers and biguanides and their polymers, the formulation having an osmolality of 225 mOsm/kg to 400 mOsm/kg.

8. The use of claim 7, wherein the tertiary amine oxide is myristamine oxide or lauramine oxide.

9. The use of claims 7 or 8, wherein the tertiary amine oxide and polylysine is used to preserve the pharmaceutical formulation.

10. The use of any one of claims 7 to 9, wherein the pharmaceutical active agent present in the pharmaceutical formulation provides for the treatment of an ocular disease or disorder.

## Patentansprüche

1. Eine topische pharmazeutische Formulierung umfassend: 0.5 ppm bis 60 ppm eines tertiären Aminoxids der allgemeinen Formel I 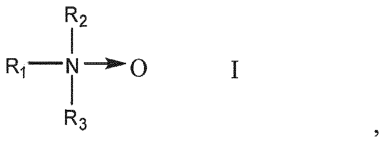 wobei R₁ ein C₈-C₁₈-Alkylrest, und R₂ und R₃ unabhängig ausgewählte C₁-C₄-Alkylreste oder C₁-C₄-Hydroxyalkylreste sind; ein pharmazeutischer Wirkstoff, wobei das tertiäre Aminoxid als Konservierungsmittel wirkt oder die konservierende Wirkung einer pharmazeutischen Formulierung verbessert, und eine zusätzliche konservierende Komponente, wobei die konservierende Komponente eine kationische, konservierende Komponente, ausgewählt aus der Gruppe bestehend aus quartären Ammoniumverbindungen und ihren Polymeren und Biguaniden und ihren Polymeren ist, wobei die Formulierung eine konservierte ophthalmische Zusammensetzung mit einer Osmolalität zwischen 225 mOsm/kg und 400 mOsm/kg ist.

2. Die Formulierung aus Anspruch 1, wobei das tertiäre Aminoxid Myristaminoxid oder Lauraminoxid ist.

3. Die Formulierung aus Anspruch 1 oder 2, weiterhin umfassend eine Komfortkomponente, ausgewählt aus der Gruppe bestehend aus Hydroxypropylguar, Hyaluronsäure, Alginat, Dexpanthenol und Hydroxypropylmethylcellulose.

4. Die Formulierung aus Anspruch 1, wobei die kationische, konservierende Komponente ausgewählt ist aus Poly(hexamethylenbiguanid), das zwischen 0.1 ppm und 1.3 ppm in der Formulierung vorhanden ist, Alexidin, das zwischen 0.5 ppm und 5 ppm in der Formulierung vorhanden ist oder α-[4-Tris(2-hydroxyethyl)-ammoniumchlorid-2-butenyl]poly[1-dimethylammonium-chlorid-2-butenyl]-ω-tris(2-hydroxyethyl)ammoniumchlorid (Polyquaternium-1), das zwischen 0.5 ppm und 3 ppm in der Formulierung vorhanden ist, oder Polyquaternium-42.

5. Die Formulierung aus einem der Ansprüche 1, 3 oder 4, wobei die konservierende Komponente Polylysin ist.

6. Die Formulierung aus einem der Ansprüche 1 bis 5, wobei der pharmazeutische Wirkstoff ein verschreibungspflichtiger pharmazeutischer Wirkstoff ist.

7. Die Verwendung eines tertiären Aminoxids der allgemeinen Formel I zur Konservierung einer topischen ophthalmischen Formulierung, die einen pharmazeutischen Wirkstoff und eine zusätzliche konservierende Komponente beinhaltet 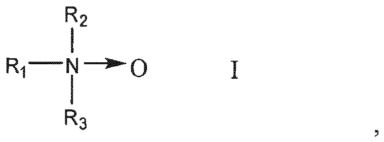 wobei R₁ ein C₈-C₁₈-Alkylrest, und R₂ und R₃ unabhängig ausgewählte C₁-C₄-Alkylreste oder C₁-C₄-Hydroxyalkylreste sind, das tertiäre Aminoxid von 0.5 ppm bis 60 ppm enthalten ist, die konservierende Komponente eine kationische, konservierende Komponente, ausgewählt aus der Gruppe bestehend aus quartären Ammoniumverbindungen und ihren Polymeren und Biguaniden und ihren Polymeren ist, die Osmolalität zwischen 225 mOsm/kg und 400 mOsm/kg beträgt.

8. Die Verwendung aus Anspruch 7, wobei das tertiäre Aminoxid Myristaminoxid oder Lauraminoxid einschließt.

9. Die Verwendung aus Anspruch 7 oder 8, wobei das tertiäre Aminoxid und Polylysin verwendet werden um die pharmazeutische Formulierung zu konservieren.

10. Die Verwendung nach einem der Ansprüche 7 bis 9, wobei der in der pharmazeutischen Formulierung enthaltene pharmazeutische Wirkstoff zur Behandlung von Augenerkrankungen oder Augenfunktionsstörungen dient.

## Revendications

1. Formulation pharmaceutique topique comprenant : 0,5 ppm à 60 ppm d'oxyde d'amine tertiaire de formule générale I 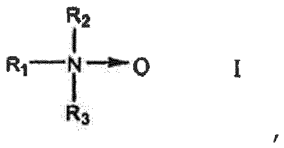 dans laquelle R₁ est un alkyle en C₈-C₁₈, et R₂ et R₃ sont indépendamment sélectionnés parmi un alkyle en C₁-C₄ ou un hydroxyalkyle en C₁-C₄ ; un agent actif pharmaceutique, dans lequel l'oxyde d'amine tertiaire fonctionne comme conservateur ou améliore l'efficacité de conservation de la formulation pharmaceutique, et un composant conservateur additionnel, dans lequel le composant conservateur est un composant conservateur cationique sélectionné parmi le groupe constitué de composés d'ammonium quaternaire et de leurs polymères et de biguanides et de leurs polymères, dans laquelle la formulation est une composition ophtalmique avec conservateur possédant une osmolalité de 225 mOsm/kg à 400 mOsm/kg.

2. Formulation selon la revendication 1, dans laquelle l'oxyde d'amine tertiaire comprend de l'oxyde de myristamine ou de l'oxyde de lauramine.

3. Formulation selon les revendications 1 ou 2, comprenant en outre un composant de confort sélectionné parmi le groupe constitué de guar hydroxypropylé, d'acide hyaluronique, d'alginate, de dexpanthénol et d'hydroxypropylméthylcellulose.

4. Formulation selon la revendication 1, dans laquelle le composant conservateur cationique est sélectionné parmi du poly(hexyaméthylène biguanide), qui est présent dans la formulation de 0,1 ppm à 1,3 ppm, de l'alexidine, qui est présente dans la formulation de 0,5 ppm à 5 ppm, ou du α-[4-tris(2-hydroxyéthyl)-chlorure d'ammonium-2-butényl]poly[1-chlorure de diméthylammonium-2-butényl]-ω-tris(2-hydroxyéthyl)chlorure d'ammonium (polyquaternium-1), qui est présent dans la formulation de 0,5 ppm à 3 ppm, ou du polyquaternium-42.

5. Formulation selon l'une quelconque des revendications 1, 3 et 4, dans laquelle le composant conservateur est la polylysine.

6. Formulation selon l'une quelconque des revendications 1 à 5, dans laquelle l'agent pharmaceutique est un agent pharmaceutique de prescription.

7. Utilisation d'un oxyde d'amine tertiaire de formule générale I pour contribuer à la conservation d'une formulation ophtalmique topique qui comprend un agent pharmaceutique et un composant conservateur supplémentaire 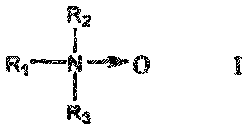 dans laquelle R₁ est un alkyle en C₈-C₁₈, et R₂ et R₃ sont indépendamment sélectionnés parmi un alkyle en C₁-C₄ ou un hydroxyalkyle en C₁-C₄, l'oxyde d'amine tertiaire est présent de 0,5 ppm à 60 ppm, le composant conservateur est un composant conservateur cationique sélectionné parmi le groupe constitué de composés d'ammonium quaternaire et leurs polymères et de biguanides et leurs polymères, la formulation possédant une osmolalité de 225 mOsm/kg à 400 mOsm/kg.

8. Utilisation selon la revendication 7, dans laquelle l'oxyde d'aminé tertiaire est de l'oxyde de myristamine ou de l'oxyde de lauramine.

9. Utilisation selon la revendication 7 ou la revendication 8, dans laquelle l'oxyde d'amine tertiaire et la polylysine sont utilisés pour conserver la formulation pharmaceutique.

10. Utilisation selon l'une quelconque des revendications 7 à 9, dans laquelle l'agent pharmaceutique actif présent dans la formulation pharmaceutique permet le traitement d'une maladie ou d'un trouble oculaire.
